# EUROPEAN PATENT APPLICATION

(11) **EP 3 885 736 A1**
(43) Date of publication of application: **29.09.2021**
(21) Application number: 20166006.5
(22) Date of filing: 26.03.2020
(51) Int. Cl.: G01N 1/02, A61B 10/02, A61H 19/00, G01N 33/487

(54) **SEX TOY ADAPTED TO COLLECT BODY FLUID AND/OR CELLS FOR DIAGNOSTIC TESTING**

(71) Applicant: Dieterle, Frank, 4102 Binningen (CH); Stübinger, Stefan, 4102 Binningen (CH)
(72) Inventor: Dieterle, Frank, 4102 Binningen (CH); Stübinger, Stefan, 4102 Binningen (CH)
(74) Representative: Specht, Peter

(57) **Abstract**

A sex toy (1) adapted to collect a body fluid and/or cells as present in a genital, especially in a female vagina, for a diagnostic test, especially an in-vitro diagnostic test and/or a biomarker test, comprising a housing (2), wherein the housing (2) comprises at least one sampling port (3) leading into at least one sampling channel (4) for introducing a sample of body fluid and/or cells through the sampling port (3) and said sampling channel (4) inside said housing (2); and an assembly comprising said sex toy (1) and a docking station and a method for analysing diagnostic tests of body fluid and/or cells.

## Description

The current invention relates to a sex toy, more particularly to a sex toy adapted to collect a sample of a body fluid and/or cells through one or more ports and channels in said sex toy. A test module integrated in the sex toy or as in an external station can analyse said body fluid and/or cells enables the measurement of general health and/or lifestyle parameters as well as to operate diagnostic testing of parameters with medical implications.

According to WHO Europe Feb 2020 simple and cost-effective preventive and curative interventions are underused.

Reports from research in India (SAGE journals Feb 2017) and Saudi Arabia (SAGE journals June 2019) amongst others regarding the importance of early identification of health issues and diseases reveal, there is still a huge gap between knowledge and practices towards routine medical check-up globally.

The most contributing feedback factors for avoiding routine medical check-up are lack of time, laziness, uncongenial situation, cost intensive and unavailability of medical centres in remote and rural areas.

The collection, management and combination of health data from different sources give consumers and patients, health professionals and medical centres an all-inclusive data knowledge to support the diagnosis and treatment of patients.

These data can trigger interactions and assist health professionals in the decision-making regarding diagnosis and treatment, especially in cases of the detection of a new disease.

It has been observed that there is the change in consumer and patient attitudes and behaviors towards greater engagement in health and health management.

There is an increasing affinity for new technologies commonly known as smart devices, a smartwatch for example, enabling consumers and patients to gain control over health and lifestyle issues without the need to consult health professionals.

However, where these devices are only measuring peripheral physical parameters such as pulse, heartbeat and temperature for example, in- depth diagnostic tests are not yet available with such new gadgets.

Diagnostic devices for the analysis of salvia are well known in the art. However, salvia has other components as body liquids from the genital such as vaginal blood, semen or the like. The analysis of these liquids is far more significant for several diseases.

Sex toys have accompanied human history ever since. Vibrating devices intended for sexual gratification by artificial means are well known to the art (US20100087703A1).

During the period starting in the late 19^{th} century up until the sexual revolution of the 1960s and '70s, inventors and distributers of sex toys in general and/or vibrating devices in specific have claimed supposedly medical use. Normally the sex toys are designed due to hygienic requirements in a way that body fluids, such as blood, cells and semen, will not be introduced inside the housing.

A lot of households possess a sex toy. A use of a sex toy in a medical application is so far not known.

It is one object of the current invention to use said sex toy at least for the sampling of body fluids, such as blood or body fluids, and/or cells.

It is a further object of this invention to provide an affordable and simple to use device to provide a non-invasive testing procedure, to support early monitoring and diagnosis, and to encourage consumers and patients to carry out regular self-testing at home.

The present invention incorporates the genuine approach of combining sexual gratification with a valuable implementation enabling any user - regardless of any knowledge about diagnostic processes or procedures - to perform a diagnosis of their own body fluid and/or cells in private and without the effort of taking a sample of the same to a medical professional.

It is a further object of the invention to provide an appliance for self-measuring general health and lifestyle parameters as well as diagnostic parameters with medical implications in-depth with no need of sampling at medical centres.

A further object of this invention is to integrate technology available in a simple to use device in order to make a data matching with data from tests performed at home possible.

An inventive sex toy is adapted to collect a body fluid and/or cells present in a genital, such as blood present in a female vagina or semen present in a male penis, for a diagnostic test, comprises a housing with a sampling port leading into a sampling channel for collecting a sample of body fluid and/or cells.

The sex toy may have various forms. Most common might be the rod-shaped form of a dildo. Also, spherical shapes are known as well as cup-shaped sex toys for men. Partner toys might have various shapes, such as u-shaped forms and the like. Therefore, the sex toy is not limited to any specific form. The Sex toy can be used as a stand-alone toy with all necessary diagnostic equipment integrated in the sex toy or in an alternative embodiment the sex toy can be used together with a docking station.

A high number of the sex toys are provided with a vibration mechanism for the massage of the genital. Vibration can be done by various mechanical elements such as motor-driven elements. Other sex toys have no vibration mechanism and are used by manual movement. The current invention is not limited to a specific massage mechanism of said sex toy. A sex toy however should not be interpreted by unintended uses of other household appliances, such as shower heads.

The sample of body fluid and/or cells can consist of either vaginal fluid, vaginal blood, menstrual blood, vaginal cells or a combination of two or more components of the same or one of the same.

It will be collected whilst in contact with the inside of a vagina or in contact with the penis. The flow and/or collection can be triggered by way of capillary force or by an aspiration mechanism.

Optional one and/or more pumps could be applied to advantageously force the flow of sample actively to achieve a faster sampling.

According to invention the housing can be fitted with one or more sampling ports and corresponding sampling channels preferably at different positions of the housing. This way a sample can be drawn from the genital.

For example, a sampling channel at the tip of the housing could facilitate the sampling close to the cervix. This could be beneficial as it may sample a different component of body fluid and/or cells.

Multiple sampling ports and corresponding sampling channels could also lead to a faster and/or more homogenous sampling, which is of advantage.

The sampling process is meant for one and/or more diagnostic tests of one or more samples. The test can be either an in-vitro diagnostic test, defined as a method, or a biomarker test or both.

The latter is also called "molecular testing" or "genetic testing." This test can detect cancer for example.

The list of analytes in a sample collected which could be measured in one and/or more samples by way of a diagnostic test could comprise at least one of the ingredients in and/or properties of the sample:
Proteins 1, 2
Peptides 3, 4
Antibodies 5
small molecules 6
DNA 7
mRNA 8
endogenous metabolites 9, 10
drugs 11
drug metabolites 12
drug components and additives
therapeutic antibody 13
cells 14
cell counts 15
ions and salts 16
mutations of nucleic acids 17
physical properties, e.g. ph, pressure (as measure of muscle strength), temperature, humidity 18
amount of cells and sperms and sperm mobility
blood gases 19, 20

Information to perform suitable diagnostic tests for the aforementioned parameters can be found in the following references:
1 https://doi.org/10.1016/i.iscs.2014.09.001
2 Journal of Natural Medicines (2018) 72:32-42_ https://doi.org/10.1007/s11418-017-1144-z
3 VECTOR-BORNE AND ZOONOTIC DISEASES Volume 12, Number 5, 2012aMary Ann Liebert, Inc. DOI: 10.1089/vbz.2011.0779
4 Current Medicinal Chemistry 14(5):531-46 (DOI: 10.2174/092986707780059698)
5 https://www.ncbi.nlm.nih.gov/pmc/articles/PMC3614608/pdf/IJBS-2-217.pdf
6 https://www.ncbi.nlm.nih.gov/pmc/articles/PMC3962833/pdf/nihms556498.p df
7 https://www.ncbi.nlm.nih.gov/pmc/articles/PMC5770625/
8 https://www.ncbi.nlm.nih.gov/pmc/articles/PMC5944930/pdf/pone.0196438. pdf
9 https://www.ncbi.nlm.nih.gov/pmv/articles/PMC5538621/
10 https://www.ncbi.nlm.nih.gov/pubmed/31797630
11 Ther Drug Monit. 2016 Apr;38(2):143-69. Doi: 10.1097/FTD.0000000000000260.
12 Ther Drug Monit. 2016 Apr;38(2):143-69. Doi: 10.1097/FTD.0000000000000260.
13 The AAPS Journal, Vol. 18, No. 1, January 2016 (# 2015) (DOI: 10.1208/s12248-015-9821-x)
14 Clinics in Laboratory Medicine 35(1 ):1-10 (DOI: 10.1016/j.cll.2014.11.003)
15 Clinics in Laboratory Medicine 35(1):1-10 (DOI: 10.1016/j.cll.2014.11.003)
16 Abbott I-Stat 1 System (https://www.pointofcare.abbott/int/en/offerings/istat/istat-handheld)
17 World J Gastroenterol 2016 November 21; 22(43): 9604-9612
18 https://support.apple.com/en-us/HT208955
19 Abbott I-Stat 1 System (https://www.pointofcare.abbott/int/en/offerings/istat/istat-handheld)
20 Hum Reprod Update. 2017 Nov 1;23(6):646-659. Doi: 10.1093//humupd/dmx022.

The opportunities which can be analyzed extend physical properties that can be measured with state of the art devices, such as an electronic watch, known as a smartwatch.

The sex toy is able to measure ingredients and/or parameters and therefore advantageously covers support for users to monitor and/or manage their lifestyle.

For example, tests with biomarkers monitoring the impact of stress, diet, nutrition and aging or one of those can support the user in lifestyle management and are well known in the art. An example for a life-style marker (e.g. oxidative stress) can be found under the following reference: https://www.ncbi.nlm.nih.gov/pmc/articles/PMC2039729/

The list indicates that the diagnostic test will allow diagnosing different diseases similar to blood-based tests.

For example, the diagnosis of sexually transmittable diseases could be of particular interest to a user, as it usually requires sampling with swaps performed by gynecologists. This could be a very sensitive situation that could cause uncongenial stress.

This could mean that a person having suspicion of sexually transmittable diseases might avoid consulting a medical professional until symptoms get worse.

This could not only be of risk for the person but may also have a massive financial impact for the society due to increased costs for treatment for example.

The invention enables anyone to privately screen themselves and only visit health professionals if the need for medical services is confirmed.

The invention allows the user to diagnose these diseases in private, at home for example, without the need to attend a physician.

Further diseases of potential interest to a user to be diagnosed are different types of cancer such as endometrial cancer, cervical cancer or vaginal cancer.

In principle, any disease can be diagnosed and/or screened and/or monitored, for which relevant biomarkers in vaginal fluid and/or menstrual blood and/or vaginal cells can be found and for which specific types of tests for the presented device have been or will be developed and made available.

This is not only beneficial to an individual user but to communities globally, especially to those underserved with medical support.

Since patients can test themselves the diagnostic device allows the presence of health professionals to be directed to critical medical activities.

Self-testing also means that the device protects health professionals from exposure to a safety hazard when taking blood samples or vaginal swaps from patients.

The key value proposition of the invention is the possibility to collect a sample in private, at home for example, screen for diseases, diagnose and monitor different diseases without the need to book an appointment with a health professional or medical centre, first to have taken blood samples and potentially second to discuss the test result, which can be very time consuming altogether.

The invention promotes self-sampling and enables self-testing.

Overall, this arrangement has multiple advantages. In addition to before mentioned ones the invention will achieve further improvements and benefits on different levels. In essence:
An increase of self-testing will have a positive impact on communal budgets worldwide.

Self-testing will also positively influence the balance between knowledge about the need for preventive and curative intervention and practice of doing so. Self-testing will action knowledge about the need for early preventive measures.

Self-testing can be delivered to developing countries where detection and control of infectious diseases is a major problem, partly due to the fact that low and middle income countries usually struggle to provide sufficient healthcare services access to their population. Self-testing may have a positive impact for change.

Particular embodiments and other and further objects of the invention will be pointed out or indicated in the dependent claims hereinafter or will be apparent to one skilled in the art upon an understanding of the invention or implementation of it in practise.

The housing might have an opening to one side through which a support unit which could preferably be introduced through the opening inside the housing. The support unit can preferably be slid into the housing. The support unit can be provided with an end cap, that forms a fluid-tight sealing with the housing when the support unit is introduced in the housing.

The in one preferred embodiment of the invention, the support unit might comprise a test module can be non-exchangeable integrated in the sex toy. Some diagnostic tests can be performed in the test modules, such as the measurement of physical properties. For other diagnostic tests the sample can be transferred to a station, such as a docking station, where other diagnostic tests are performed.

A preferred embodiment would be that the test module would be built modular and replaceable. In this way different cartridges for a variation of diagnostic tests could be inserted and after use replaced with new ones of the same and/or a different analytical target profile (ATP).

In another embodiment parts of the test module might be modular and replaceable, the first analyzing unit for example whilst other parts of the test module may remain.

This first analyzing unit can be a component of the test module for carrying out a diagnostic test.

The housing can be shaped cylindric with a spherical front cap. The shape could also be in any other form to function as a sex toy, elongated curved for example.

Different types of diagnostic tests could be implemented, in particular a lateral flow test, known for being simple, economic and quick to perform as they generally show results in around 5-30 minutes.

Other tests may be carried out, such as
vertical flow tests
dip-stick tests
sensor systems
immunoassays
color reaction tests
clinical chemistry tests
nucleic acid tests
protein tests
metabolic test
genetic tests
molecular tests
LAMP tests
paper based tests
chromatographic tests
electrochemical tests, e.g. glucose measurements and/or
physiologic and/or physical properties, e.g. ph and/or pressure as measure of muscle strength and/or temperature and/or humidity

In this way testing can be chosen for specific demands which is of great advantage as target-oriented testing will ultimately trigger goal-specific consultation and action.

As semen contains the same analytes as vaginal body fluid and/or cells does, an advantageous embodiment would be to provide a device to encourage and enable male self-testing for the same reasons applied to females.

Instead of using an elongated cylindric shaped housing it could be of advantage to provide a shape of the housing mirroring a female vagina for example, for the provision of male gratification.

The scope of such implementation would be to provide opportunity to male to sample semen for a diagnostic test equivalent to proposed device for female usage. The following examples may illustrate further some specific embodiments and aspects of the invention. The invention is not limited to these use cases, which are listed for illustration purposes only.

### Sexually Transmittable Diseases

The diagnosis of sexually transmittable disease (STDs), such as gonorrhea, syphilis, chlamydia or trichomoniasis is of high interest. In many cases infections are asymptomatic and will not be diagnosed. If STDs are not treated, further complications can occur, such as vaginal bleeding, fever, infertility, pelvic inflammatory disease, pre-term delivery, passing the diseases to the baby, impacting the health of babies and many more.

The current diagnosis of STDs require blood testing (syphilis) or intracervical swaps (gonorrhea and chlamydia), which only a physician or trained health worker can perform.

The disclosed diagnostic device will allow to diagnose one and/or several sexually transmittable diseases based on cells, vaginal fluid, vaginal blood or menstrual blood, using for example immunoassays or molecular technologies.

The device will allow women to run screening tests without the need for visiting a physician to perform tests of vaginal and intracervical swaps or blood testing.

### Bacterial vaginosis

Bacterial vaginosis (BV) is a disease of the vagina caused by excessive growth of bacteria. Common symptoms include increased vaginal discharge, while in up to 50% of cases there may be no symptoms. Bacterial vaginosis doubles the risk of infection by a number of other sexually transmitted infections, including HIV and increases the risk of early delivery among pregnant women. Standard of care diagnosis is based on vaginal swaps and treatment is based on administration of antibiotics.

An implementation of the disclosed device allows the early diagnosis of Bacterial vaginosis without the need to attend a gynecologist for performing a diagnosis based on vaginal swaps.

### Pelvic Inflammatory Diseases (PID)

Pelvic inflammatory disease (PID) is an infection of the upper part of the female reproductive system, namely the uterus, fallopian tubes, and ovaries, and inside of the pelvis. Often, there may be no symptoms. The disease is caused by bacteria. PID can cause scarring inside the reproductive system, which can later cause serious complications, including chronic pelvic pain, infertility, ectopic pregnancy, the leading cause of pregnancy-related deaths in adult females, and other complications of pregnancy.
An implementation of the disclosed device allows the early screening for PID based on CRP levels, nucleic tests or additional biomarkers and thus recommends to consult a physician for further diagnosis and treatment, e.g. antibiotics.

### Endometriosis

Endometriosis is a condition in which cells similar to those in the endometrium, the layer of tissue that normally covers the inside of the uterus, grow outside of it. Symptoms include chronic pelvic pain, while 25% of cases are symptom-free.

An implementation of the disclosed device allows the early screening for endometriosis based on biomarkers associated with endometriosis, e.g. CA-125. Upon positive test results the devices recommends to visit a physician for further diagnosis and treatment, e.g. hormone therapy.

### Vaginal Yeast (candidal vulvovaginitis)

Vaginal yeast infection, also known as candidal vulvovaginitis and vaginal thrush, is excessive growth of yeast in the vagina that results in irritation. The most common symptom is vaginal itching, which may be severe.

An implementation of the disclosed device allows the self-diagnosis of vaginal yeast infection based on the detection of antigens in the vaginal fluid. Upon positive test results treatment can be started.

### HIV

The human immunodeficiency viruses (HIV) are two species of Lentivirus (a subgroup of retrovirus) that infect humans. Over time they cause acquired immunodeficiency syndrome (AIDS), a condition in which progressive failure of the immune system allows life-threatening opportunistic infections and cancers to thrive. Without treatment, average survival time after infection with HIV is estimated to be 9 to 11 years.

An implementation of the disclosed device allows the early screening and diagnosis of HIV based on antibodies for HIV-1 and HIV-2. Upon positive test results the device recommends to visit a physician for further diagnosis and treatment.

### Preterm Delivery

Preterm delivery is the leading cause of neonatal death.

An implementation of the disclosed device allows the self-diagnosis of an increased risk of pre-term delivery based on biomarkers such as fetal fibronectin or placental alpha microglobulin. Upon positive test results preventive measures and a change of lifestyle can be started.

### Cancer Screening

Different forms of cancer, such as cervical cancer, endometrial cancer, ovarian cancer or breast cancer, just to mention some forms of cancer are the leading cause of death for women. When detected early most forms of cancer can be treated today.

Some implementations of the device allow to screen for the risk of being affected by specific forms of cancer, for example based on the genes of BRCA1 or BRCA2 genes.

### Infective Diseases and Tropical Diseases

Screening and diagnosis of many tropical infectious diseases is often based on the detection of antibodies against the disease or of antigens from the virus or parasite itself. While not all diseases are curable, the early detection allows the early initiation of therapy to prevent further damage. However, patients with early stages of chronic diseases or slowly progressing diseases do often not show symptoms and thus do not seek for professional healthcare advice.

Implementations of the device will enable the user for a painless early screening and detection of different infectious diseases early on and upon positive test results seek for professional help. Implementations will allow to detect single or several diseases at once ("Panel"), based on biomarkers associated with the diseases, e.g. antigens, analytes, nucleic acids. Examples are different forms of hepatitis (A, B, C, E), tuberculosis, malaria, dengue, chagas, leishmaniasis, leptospirosis, zika, human African trypanosomiasis (HAT), Japanese encephalitis, chikungunya, lymphatic filariasis, onchocerciasis, hantavirus and more.

### Preeclampsia

Pre-eclampsia (PE) is a disorder of pregnancy characterized by the onset of high blood pressure and often a significant amount of protein in the urine. In severe disease there may be red blood cell breakdown, a low blood platelet count, impaired liver function, kidney dysfunction, swelling, shortness of breath due to fluid in the lungs, or visual disturbances. Pre-eclampsia increases the risk of poor outcomes for both the mother and the baby.

The Implementation of disclosed device allows an early detection of the risk of PE based on associated biomarkers such as placental growth factor (PIGF), soluble Feline McDonough Sarcoma-(fms-) like tyrosine kinase-1 (sFlt-1), asymmetric dimethylarginine (ADMA), and methyl-lysine and thus early changes of lifestyle and treatment.

### Diabetes mellitus (DM)

Diabetes mellitus (DM) affects world-wide more than 400 million people. A close management of insulin levels is crucial for preventing complications and organ damage.

Implementations of disclosed device allow painless monitoring of glucose levels and glycated hemoglobin (HbA1c) levels to support the management of diabetes.

### Hormones

Implementations of disclosed device allow the management of fertility and menopause by measuring different hormones, such as progesterone, follicular stimulation hormone (FSH), luteinizing hormone (LH) or estradiol as single tests or several hormones combined as panel.

### Therapeutic Drug Monitoring

Many drugs in the area of neurosciences, transplantation and antibiotics require frequent monitoring of drug levels and adjustment of drug levels to find the right balance between side effects (too high drug levels) and being not efficacious (too low drug levels).

Implementations of disclosed device allow the monitoring of levels of one and/or several drugs and thus a tight management of the drug levels.

### Semen

Implementations or our disclosed device allow the characterization of semen in the vagina. The analysis of components of semen, such as proteins or DNA allow a profiling of the originator of the semen, which may be of relevance in certain situations, e.g. rape.

### Vitamins, Minerals and Nutrition

Implementations of disclosed device allow to monitor levels of different vitamins, for example vitamins A, D, E, B 12, and different minerals, for example calcium zinc and iron and thus allow the women to adjust her diet to have balanced levels of the corresponding analytes.

### Fatigue Panel

Implementations of disclosed device allow screening and diagnosing different diseases causing fatigue such as vitamin D deficiency, iron deficiency or hypothyroidism by measuring associated analytes such as 25(OH)D levels, thyroid stimulating hormone (TSH) levels or ferritin levels.

The listing above is meant to be a list of examples for which specific embodiments and aspect of presented arrangement may apply. The combination of features described by the embodiments shall not be understood as a limitation in any way.

The lateral flow test's core elements may be a cellulose based test stripe with conjugates and antibodies for example.

The readout of the test components could be performed optically, i.e. color bands of lateral flow tests evaluated visually.

Readouts could also be performed via LEDs and/or LCDs and/or, alternatively with an external device, such as a camera of a mobile phone for example.

Another readout could be achieved by radio-transmission with Bluetooth or near field technology.

Readouts with WIFI and 2g, 3g, 4g or 5g may apply.

Advantageously beforementioned options give choice to the user and their individual needs.

Test readouts could be qualitative, semi-quantitative or quantitative. The latter for example to measure the intensity of the test line to determine the quantity of analyte in the sample.

In a preferred embodiment diagnostic test component could be specifically optimized for the first analyzing unit. This could also be applied for the station.

For example, lateral flow tests can be sealed for a robust measurement. The test components can be mounted within a container or can be mounted externally.

The test components can be built-in as a fixed component or can be mounted and/or exchanged by the user. The test components might be operated by built-in components to control the testing logic or externally by an application (App) or an external device.

The test components could measure different analytes such as mentioned before.

In one of the preferred embodiments the test module could comprise a reservoir for liquids and reagents.

The test module could also comprise several reservoirs, each for different liquids and/or reagents for example.

It is of advantage to provide such reservoirs as it allows initiating different reactions in sequence, e.g. several reservoirs with several push-buttons, to be pressed in a pre-defined sequence.

The flow of such liquids and/or reagents can be by capillary force.

Preferred examples of these liquids and reagents are at least one or more of anti-coagulant agents
extraction reagents
diluents
antibodies
antigens
primers
reagents for cell disruption
BSA
aptamers
reagents for generating plasma and serum
reagents to run molecular tests e.g. PCR and LAMP and other tests
reagents to run immunoassays and colorimetric assays
or combinations therefrom

The reservoir can be exchanged with the test module. If the test module is cannot be exchanged, the reservoir can be refilled in a station, such as a docking station.

The device can provide the possibility of testing different analytes at once, for example executing panels of tests of the same analyte, e.g. proteins or of different analytes, e.g. proteins or nucleic acids.

The device and/or the test component, for example a lateral flow test stripe, may process the results internally and/or externally, for example calculating a score.

Multiple tests can be implemented via a single test component, and/or with the help of several test components.

To another advantage is the embodiment where the test module comprises a reaction chamber.

The sample could be exposed to different processes and/or reactions, for example binding reactions and/or incubations steps and/or thermocycling steps, the latter for polymerase chain reactions for example.

Further exposure could be to a Loop-mediated isothermal amplification (LAMP) of DNA for example, which is a low-cost alternative to detect certain diseases.

In addition, the exposure could comprise color reactions and/or enzymatic reactions and/or chromatography to run lateral and/or vertical flow tests and/or other protein and/or DNA separation techniques.

There could be more than one reaction chamber.

It is of advantage if the sex toy comprises a filter to filter particles of body fluid and/or cells of specific sizes for example when being introduced to the reaction chamber.

There could be more than one filter. The filter could consist of either porous membranes, sieves and nets or a combination of those.

Of advantage would be if the sex toy would comprise at least one temperature control device to adjust and control the temperature inside the reaction chamber. This could for example be a thermo element to adjust and/or control temperatures, for example during thermocycling steps.

In a further embodiment the sex toy can also comprise a microfluidic device to exploit the physical and/or chemical properties of body fluid and/or cells at a microscale.

Microfluidics have diverse assets, for example faster reaction time and/or enhanced analytical sensitivity and/or easier automation and/or integration of lab routines in one device.

They also deliver accurate measurement and/or allow to increase the measurement resolution in given applications.

It is cheap to implement as it does not involve the use of various costly equipment.

There could be more than one microfluidic device be implemented in the sex toy.

The sex toy could also comprise a waste container for the disposal of used samples and/or liquids and/or reagents. Said waste container can be part of a replaceable test module or can be emptied and cleaned inside a docking station.

Such container could also be integrated in a test stripe element of a lateral flow test.

By this arrangement the device will be beneficially protected from contamination.

In a preferred embodiment the support unit can comprise a networking unit comprising a networking modul to operate and/or control the test module (8), and/or adapted to establish a wirelessly connection, preferably to a Wide Area Network (WAN). The networking module can be adapted particularly to provide connectivity.

This can comprise the communication of data between the test module and the transceiver unit for example, for processing the diagnostic test and/or receiving test results.

Therefore the networking unit may comprise said transceiver unit adapted to transmit and/or receive digital data, preferably wireless digital data, most preferably established and/or performed by way of WIFI and/or Bluetooth and/or nearfield communication (NFC), wherein the digital data preferably comprises at least one of picture, moving images, and/or sound.

Connectivity can also be provided for remote-controlling the sex toy as a whole.

The remote could be adapted to control and/or operate the sex toy, amongst the play function of the sex toy, particularly the process of a diagnostic test. This can include processing the data transfer of test results for a send out to external electronic devices.

The remote could be designed as a touch screen remote with symbols showing commands to be processed. This would be of advantage as it would simplify remote usage.

Another provision of connectivity can be processed to connect to the World Area Network (WAN).

The networking unit might comprise a transceiver unit adapted to transmit and/or receive digital data, in a preferred embodiment wirelessly and/or established by way of WIFI.

The wireless establishment could also be facilitated by way of Bluetooth and/or nearfield communication (NFC) and/or mobile communication such as 2g, 3g, 4g or 5g.

The purpose of the transceiver unit would be the prospect of an overall communication of digital data including test results, test commands, status information of tests, command for the sex toy functions, status information of the sex toy functions, still and moving images as well as sound or one of those.

The transceiver unit could allow to transfer test results to other electronic devices, for example to mobile phones, electronic watches, TVs, to Apps running on electronic devices, to databases or to computer programmes or to one of those.

The transfer could be performed locally within the intranet and/or globally via the internet.

The transceiver unit can be equipped with a software application (App) to support data exchange and/or interactive communication as illustrated in the case-study below. These are examples and not to be understood as a limitation in any way:
i. Send data and/or commands to the test module to control the diagnostic testing procedure. For example, the logic of running the tests - different steps executed one after the other - could be implemented in the App. In addition, further critical data required for the testing procedure, e.g. batch calibration data can be implemented in the App and can be sent to the test module. Such an implementation can dramatically reduce costs of the test module, for example for a disposable one-time-use test module.
ii. Support the evaluation of the test results. For example, in the case of an optical readout presented by the analyzing unit of the test module, the mobile phone can support the collection and/or interpretation of the results via its built-in camera, e.g. quantitating the test results, while a non-trained user might only recognize qualitative "yes / no" results; and/or calculating scores on the basis of several readouts. In case of sensor results, the electronic device and/or App will support the analysis of results, e.g. transforming raw data readouts into "yes / no" answers; staging diseases and/or monitoring disease progressions and/or calculating quantitative results;
iii. Evaluate the validity of test results. For example, photos of a QR code on the packaging of the diagnostic device, or on the diagnostic device itself, for checking the expiration date of the test module and/or the type of test being used;
iv. Create a log and/or diary of multiple consecutive test results;
v. Transfer results to databases and/or physicians and/or health workers and/or electronic health-care systems;
vi. Alert the user or a remote physician about abnormal test results;
vii. Educate the user and/or remind and/or motivate the user for adhering to a testing schedule;
viii.Advise the user on test results and/or recommend follow-up activities;
ix. Collect and/or integrate additional information, for example from previously performed tests and/or from built-in App questionnaires, to calculate risk scores and/or store and/or transfer the additional data;
x. Collect data from other diagnostic and/or medical and/or electronic devices and tests.

The transceiver unit could be operating as an electronic device equivalent to an App in combination with a mobile phone. Colloquial speaking it could be named smart device.

Thus, the transceiver could operate external of the device. In a preferred embodiment it can operate integrated in the device.

In addition, there could be a voice module made available.

The voice module could facilitate to give verbal feedback to the user on the progress, on-going steps and/or status of the testing procedure.

It could also advise the user on next steps, for example to open the device and/or look at the testing results and/or take care of testing failures.

The device could also contain a voice recognition module to listen to and/or interpret and/or execute user commands, for example initiate testing.

Further the device might be connected to home-assistants, for example such as the Amazon Alexa system, the Google Home System, the Apple Siri system, the Microsoft Cortana system, the Chinese Baidu system, or to one of those, for remote control and interactions both directions.

The support unit could comprise an energy source for energy supply to energy consuming components such as a vibration device as a massage part of the sex toy for example.

The energy source could be a batterie for example. This can be advantageously a rechargeable batterie to adhere to the ecological footprint measures.

Energy might be necessarily provided to the test module and/or its components via a circuit path.

The circuit path could be achieved with a vacuum metal deposition on components of the test module and/or test stripes to be equipped with contact ports at cut off points.

This way energy supply could restart after replacement of a test module.

A similar method could be applied for data transfer between test module and transceiver unit for example, alternatively to wireless data transfer.

In addition, the support unit can comprise an end cap designed to close and/or open the housing.

The scope getting access to the internal components of the support unit can be to replace the modular test module and/or to replace integrated batteries with recharged batteries for example.

It could also be for the purpose to have a look at test results if the results are represented optically, with lines of a lateral flow test for example.

Closing the housing with end cap can be performed by a screw mechanism for example. It could also be performed by a sliding mechanism.

The end cap may either be fitted going inside the housing or enclose the housing on the outside.

It would be of advantage if the end cap would be fitted with a seal to waterproof the device. Therefore, the support unit can have said end cap designed to fit into the opening of the housing for fluid-sealing closure between the housing (2) and said end cap.

The end cap might comprise at least one activation element, to manually operate and/or control processes of the sex toy, especially the operation of the test module and/or the vibration device.

The sex toy, especially the end cap, might also be fitted with LEDs and LCDs or one of those for signalling information. The information could be for example whether the sampling and/or the diagnostic test is in progress.

The sampling channel could be moulded integral of the sampling port of the housing, forming all of a piece. A one-piece formation might avoid seams so that this arrangement might be advantageous when it comes to cleaning and sterilisation.

Alternatively, the sampling channel can be part of the test module.

The housing might be fitted with an embedded window to have direct sight on optical read outs of diagnostic tests.

A preferred embodiment would be an assembly for analysing a diagnostic test of body fluid and/or cells comprising said sex toy and a station, such as a docking station.

Said docking station can provide diagnostic tests only or additionally to the diagnostic tests performed in the sex toy. When the sex toy is used for the sampling only, the station may perform all necessary diagnostic tests on the sample collected by the sex toy.

The station can also provide additional tests to the tests performed by the sex toy. The space for sensor elements inside the sex toy is limited due to its function. Therefore, the station can be provided with further sensor elements for a more detailed analysis of the sample.

In either way - the station is provided with an evaluation unit for evaluating the test results provided by the sex toy or by an integrated analysing unit. Said analysing unit can be provided with a test module described above inside the sex toy with a reservoir, reaction chamber, filter, waste container and one or more sensor elements and pumps.

The test module can be replaceable, for example for as a single use application.

The analysing unit may be further provided with non-replaceable sensors such as an optical sensor for the detection of the measurement results of the lateral flow test.

The sample can be transferred from the sex toy to the test module. In another embodiment the test module of the analysing unit is also the test module of the sex toy. Said test module can be transferred from the sex toy after sampling to the station.

The station could be adapted to operate and/or carry out a diagnostic test. This can comprise analysing and/or screening and/or monitoring diagnostic parameters of a sample of body fluid and/or cells.
Therefore, the station can be provided with an analysing unit.

The sample might be provided in a modular test module inserted into the port of insertion of the station.

An external analyzer can host more complex and larger equipment in comparison to the first analyzing unit comprised by the test module.

Therefore, this approach might be preferred for more complex analysis, for example gene sequencing and/or other nucleic acid analysis and/or immunoassay test.

An immunoassay test for example is a biochemical test that measures the presence or concentration of a macromolecule and/or a small molecule in a solution usually through use of an antibody and/or sometimes an antigen, which makes this testing more comprehensive.

In these cases, the functions of the modular test module might be limited to sample collection and/or sample storage and/or simple sample preparation steps.

The station can be provided and/or equipped with a wireless transceiver.

The transceiver could be adapted to transmit and/or receive digital data preferably via a Wide Area Network (WAN), wherein the digital data are in particular related to diagnostic tests.

Operating the station can be made possible via a provided App, as a computer program, in combination with a mobile phone.

Digital data to be transmitted and/or received can be diagnostic test related only.

It is of advantage when a station would have corresponding connectivity to the first analysing unit to fully make use of its function.

The port of the station can alternatively be suitable for the insertion of the sex toy. In a preferred embodiment, the sample can be discharged from the sex toy, the sex toy can then be cleaned and charged, preferably while the diagnostic tests are performed inside the station.

In a preferred embodiment the station might execute actions as listed in below case study. However, these are examples and not to be understood as a limitation in any way.
i. Run the analytical testing of the samples. For example, run immunoassay;
ii. Evaluate the validity of test results. For example, the device might check the expiration date of the diagnostic device and/or the type of test being used;
iii. Create a log and/or diary of multiple consecutive test results;
iv. Transfer results to databases and/or physicians and/or health workers and/or electronic health-care systems;
v. Alert the user and/or a remote physician about abnormal test results;
vi. Educate the user and/or remind and/or motivate the user for adhering to a testing schedule;
vii. Advise the user on test results and/or recommend follow-up activities;
viii.Collect and/or integrate additional information, for example from previously performed tests and/or from built-in App questionnaires, to calculate risk scores and/or store and/or transfer the additional data;
ix. Collect data from other diagnostic and/or medical and/or electronic devices and/or tests.

The station can comprise a control unit for manually operation and/or processing analyzing and/or testing of test modules.

There could also be a set of LEDs and/or LCDs installed for signalling information.

Advantageously the station can comprise a cleaning unit for rinsing and/or cleaning the sex toy.

It would be specifically an advantage when the cleaning unit could be equipped with a specific reach out mechanism for cleaning the sampling port and corresponding sampling channel.

The cleaning unit might be also equipped with a setup for sterilization holding a sterilization medium reservoir.

Aforementioned implementation could beneficially assist to maintain a high standard of hygiene of the sex toy.

In a further embodiment it can be of asset if the docking station could comprise a charging set for a recharge of the energy source, particularly a rechargeable batterie.

An inventive method for analysing diagnostic tests of body fluid and/or cells using a sex toy, particularly the inventive sex toy, the method comprising:
i. Providing the sex toy;
ii. Collection of a sample of body fluid and/or cells through the sampling port into the sampling channel
wherein a diagnostic test is performed by the sex toy and/or by the docking station with said collected sample.

More specifically the inventive method can be provided with one or more of the following steps
i. Providing the sex toy with a test module;
ii. Inserting the sex toy accordingly;
iii. introducing a sample of body fluid and/or cells through the sampling port into the sampling channel for collection into the reaction chamber preferably by capillary force;
iv. Starting the diagnostic test by default and/or manually using the activation element and/or an external mobile phone;
v. Waiting for diagnostic test completion signal;
vi. Removing the sex toy;
vii. Sighting the diagnostic test result through the window;
viii. Ejecting the test module out of the sex toy inserting said test module into the docking station;
ix. Executing further diagnostic testing commanding manually using control unit and/or an external mobile phone;
x. Transferring diagnostic tests to physician commanding the transceiver module with an external mobile phone.

An advantageous embodiment of an inventive sex toy is further explained in detail by a drawing. Specific parts of the embodiment can be understood as separate features that can also be realized in other embodiments of the invention. The combination of features described by the embodiment shall not be understood as a limitation of the invention:
- Fig. 1: schematic view of the device from outside;
- Fig. 2: illustrated view on sliding the support unit of the device;
- Fig. 3: schematic view on housing only;
- Fig. 4: schematic view on support unit only with sampling channel part of test module;
- Fig. 5: illustrated view on support unit only with sampling channel part of modular test module being inserted;
- Fig. 6: schematic view on support unit only with sampling channel part of housing;
- Fig. 7: illustrated view on support unit only with sampling channel part of housing, modular test module being inserted;
- Fig. 8: illustrated view on station with modular test module path;
- Fig. 9: illustrated view on device and external mobile phone.

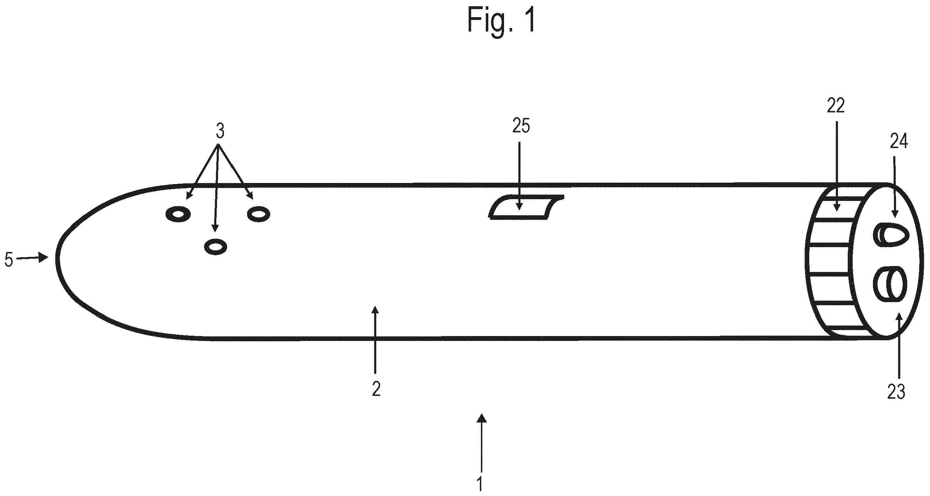

An embodiment of an inventive sex toy 1 according to the invention is shown in Fig. 1, the housing 2 shaped cylindric with a spherical front cap 5 and an end cap 22 opposite to the front cap 5.

In this further embodiment the housing 2 comprises three sampling ports 3. Corresponding sampling channels 4 are not shown in Fig. 1 due to the schematic drawing perspective.

The housing comprises a window 25 to allow sight into the housing 2 for an optical readout of a test result.

The end cap 22 closes the opening 6 of the housing 2.

To operate and/or control processes of the device manually the end cap 22 is fitted with at least one activation element 23. For signalling information, the end cap 22 is further equipped with at least one of LEDs and LCDs 24.

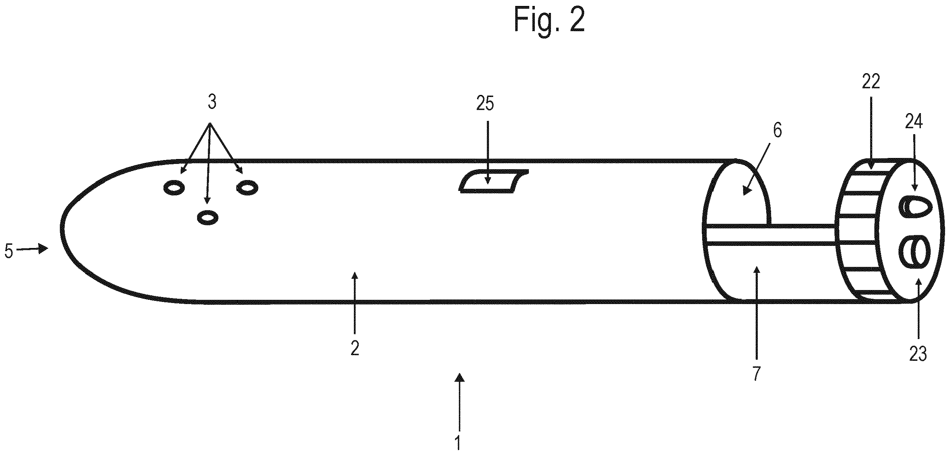

Fig. 2 reveals the support unit 7 demonstrating the sliding process being inserted and/or removed into and/or out the housing 2 through the opening 6. It also shows the half cylindrical shape of the support unit 7 corresponding to the shape of the housing 2.

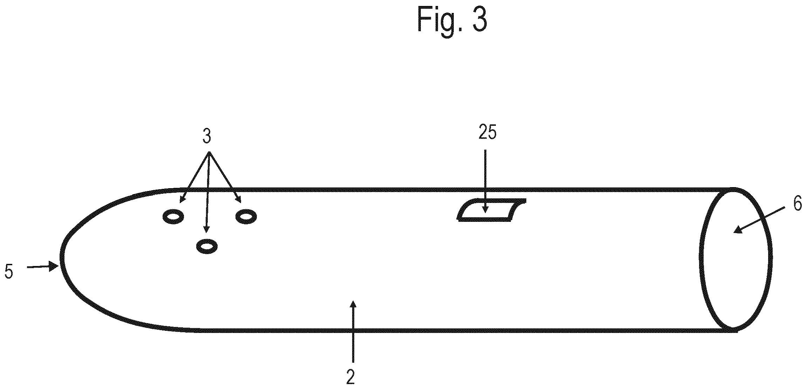

The housing 2 in Fig. 3 is shown with the open 6 as an open shaped cylinder with a spherical front cap 5. The shape of the housing 2 could also be descripted as phalloid.

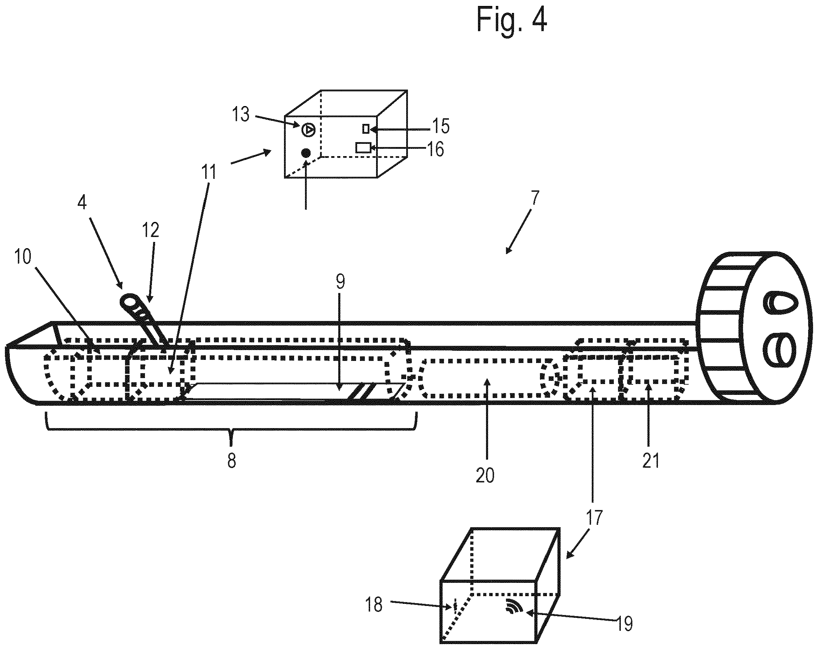

Fig 4 shows the support unit 7 comprising the test module 8. The test module 8 is build and/or equipped for analysing and/or screening and/or monitoring diagnostic parameters of collected sample body fluid and/or cells with medical implications as well as general health and lifestyle parameters.

It comprises at least a first analysing unit 9. The scope of the first analysing unit 9 is to carry out a diagnostic test, particularly to run a lateral flow test.

The test module 8 is equipped with at least one reservoir 10. The scope of the reservoir is to hold liquids and/or reagents, particularly diluents and/or extraction reagents.

Fig. 4 further shows the reaction chamber 11 with the sampling channel 4 mounted next to it showing an embodiment, where the sampling channel 4 is defined as being part of the test module 8.

The reaction chamber 11 is adapted to run different processes and/or reactions and/or separation techniques, particularly incubation steps and/or binding reactions and/or chromatography. The latter is a separation technique.

It is further shown that the reaction chamber 11 comprises at least one filter. The purpose is to filter the body fluid and/or cells being introduced through the sampling channel 4 before it enters the reaction chamber 11.

The filter is particularly a porous membrane and/or a sieve and/or a net. The scope of filtering is to filter cells or particles of specific size out of the body fluid and/or cells.

In an enhanced display detail of Fig. 4 it is shown that the reaction chamber further comprises at least one pump 13 for actively performing the sampling. This contributes to a faster sampling.

It is also shown reaction chamber 11 comprises at least one thermo element 14 to adjust and/or control temperatures.

Further it is shown the reaction chamber 11 comprises a microfluidic device 15 to exploit the physical and/or chemical properties of body fluid and/or cells at a microscale. This process enables analysing with very small volumes of fluids, down to femtoliters (fIL).

Further enhanced display detail of Fig. 4 shows that the reaction chamber 11 comprises a waste container 16 for disposal of used samples and/or liquids and/or reagents.

Fig. 4 further discloses that the support unit 7 holds a networking unit 17.

In another enhanced display detail of Fig. 4 it is revealed that the networking unit 17 comprises a networking module 18, preferably adapted to add at least one interface, preferably wireless, particularly to operate and/or control the test module 8, and/or adapted to establish a wirelessly connection, preferably to a Wide Area Network (WAN).

Further discloses enhanced display detail of Fig. 4 that the networking unit 17 also comprises a transceiver unit 19.

The transceiver unit 19 is adapted to transmit and/or receive digital data, preferably wirelessly, preferably established and/or performed by way of WIFI and/or Bluetooth and/or nearfield communication (NFC).

The digital data includes at least one of test results, test commands, status information of tests, command for the sex toy functions, status information of the sex toy functions, still images, moving images, and sound.

Fig. 4 discloses further that the support unit 7 comprises an energy source 20.

The supply for energy is for energy consuming components of the sex toy 1 as a whole and comprising the vibration device 21 to run the play part of the sex toy 1.

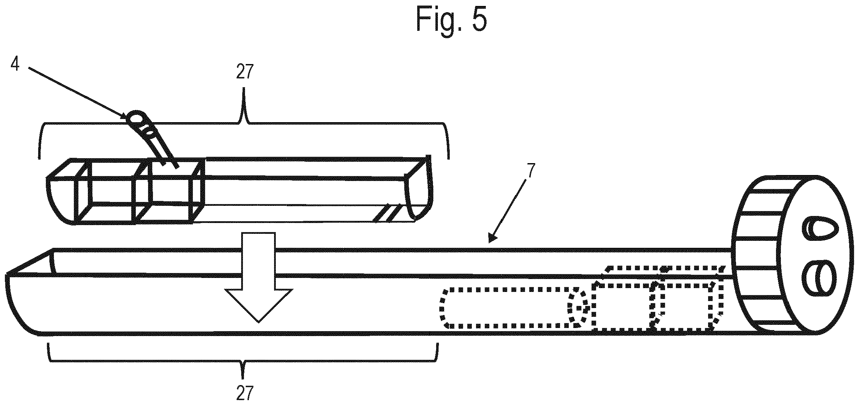

Fig. 5 illustrates the insertion of the modular test module 27 into the device, the housing 2 being removed, using a modular test module 27.

In this embodiment the modular test module 27 is built replaceable.

The implementation shows the sampling channel 4 as part of the modular test module 27.

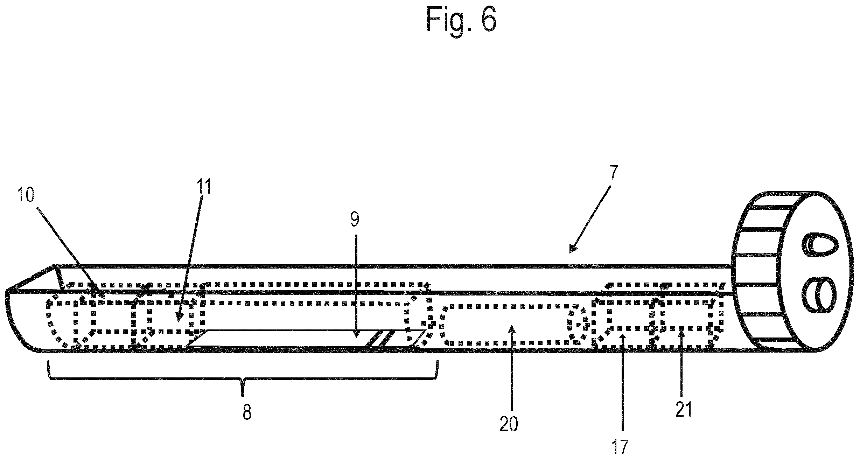

Fig. 6 discloses the support unit 7 after removing the housing 2. In this embodiment is the sampling channel 4 moulded integral of the sampling port 3 of the housing 2, as one-piece, not shown on this drawing.

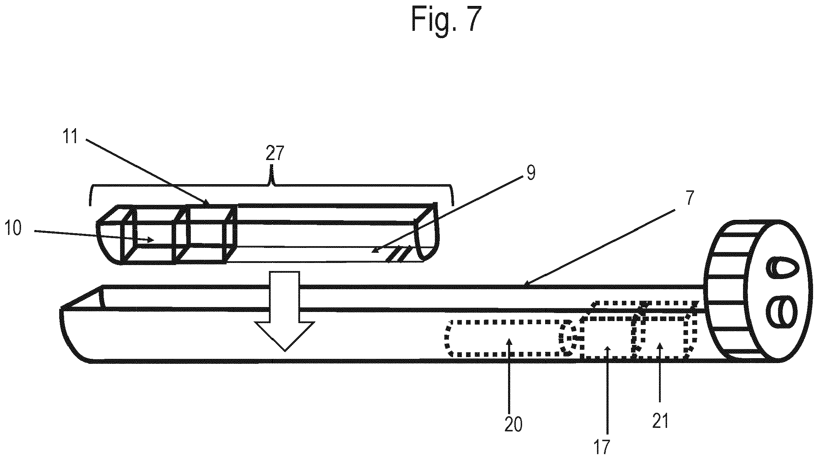

The implementation shown in Fig. 7 illustrates the insertion of the modular test module 27 into the device, the housing 2 being removed. The sampling channel 4 is moulded integral of the sampling port 3 of the housing 2, one-piece, not shown on this drawing.

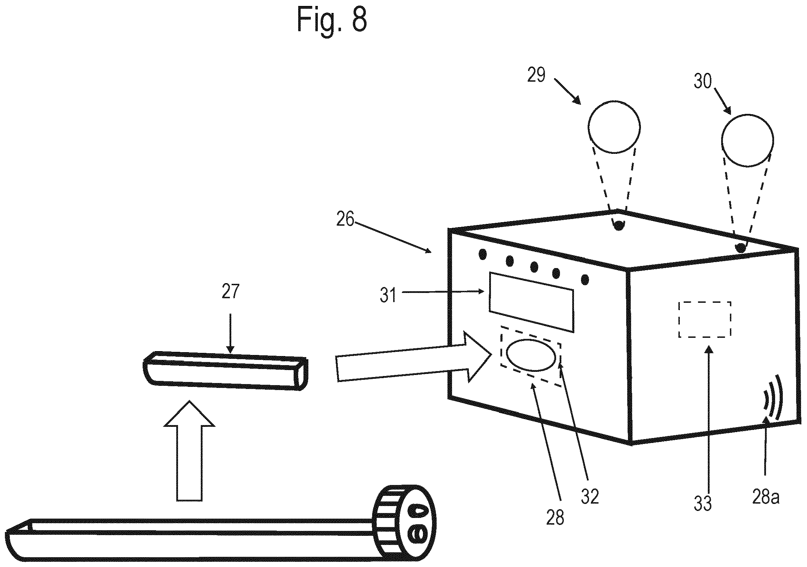

In a preferred embodiment an external docking station is provided (Fig. 8).

For running a test, the modular test module 27 will be removed from the device and inserted into the external station 26 passing the port for insertion 28 of the station 26.

The port 28 in Fig. 6 is adapted to receive the test module 27 but can be also be wider, so that it is able to receive the whole sex toy 1.

Said station 26 can be provided with an analyzing unit 32 comprising one or more sensor units, such as an optical sensor, and an evaluation unit 33 for the evaluation of the test results measured either by the station 26 or by the sex toy.

The purpose for testing a modular test module 27 in the station 26 is the logic that implementation of the station 26 provides more space for more complex components with the prospect of running more enhanced analyses.

The station 26 is also provided and/or equipped with a wireless transceiver module 28a.

The transceiver module 28a is adapted to transmit and/or receive digital data.

The data transfer will be preferably processed via a Wide Area Network (WAN) and will be in particular diagnostic test related.

Fig. 8 also shows the control unit 31 of the station, which is to operate manual.

It is also revealed in Fig. 8 that in a further preferred embodiment the station 26 comprises a cleaning unit 29.

The scope for the cleaning unit 29 is to clean the sex toy 1, especially the sampling port 3 and/or the sampling channel 4 moulded integral of the sampling port 3 of the housing 2.

Another preferred embodiment shown in Fig. 8 is that the docking station comprises a charging set 30 to recharge the energy source 20.

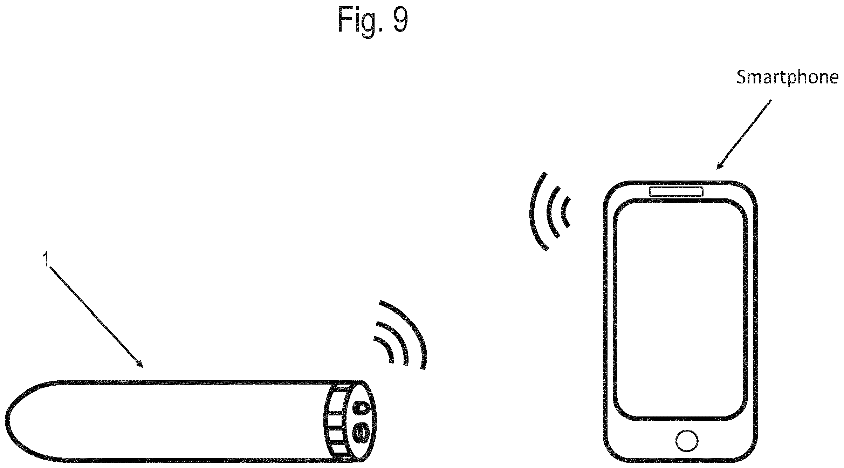

Fig.9 illustrates wireless connectivity to an external mobile phone, colloquial called smartphone.

### List of references

- 1: sex toy
- 2: housing
- 3: sampling port
- 4: sampling channel
- 5: spherical front cap
- 6: opening
- 7: support unit
- 8: test module
- 9: first analysing unit
- 10: reservoir
- 11: reaction chamber
- 12: filter
- 13: pump
- 14: thermo element
- 15: microfluidic device
- 16: waste container
- 17: networking unit
- 18: networking module
- 19: transceiver unit
- 20: energy source
- 21: vibration device
- 22: end cap
- 23: turning knob
- 24: LED/LCD
- 25: window
- 26: docking station
- 27: modular test module
- 28: port for insertion
- 28a: transceiver module
- 29: cleaning unit
- 30: charging set
- 31: control unit
- 32: analysing unit
- 33: evaluation unit

## Claims

1. Sex toy (1) adapted for the collection of a body fluid and/or cells as present in a genital, especially in a female vagina, for a diagnostic test, especially an in-vitro diagnostic test and/or a biomarker test, comprising a housing (2), wherein the housing (2) comprises at least one sampling port (3) leading into at least one sampling channel (4) for collecting a sample of body fluid and/or cells through the sampling port (3) and said sampling channel (4) inside said housing (2).

2. Sex toy (1) according to claim 1, **characterized in that** the housing (2) has an opening (6), preferably a slot, for inserting an support unit (7) into the housing (2), wherein the support unit (7) is part of said sex toy (1) and comprises at least one test module (8, 27).

3. Sex toy (1) according to claim 1 or 2, **characterized in that** the test module (8, 27) is adapted for analysing and/or screening and/or monitoring diagnostic parameters of a collected sample body fluid and/or cells with medical implications as well as general health and lifestyle parameters, comprising at least a first analysing unit (9) for carrying out a diagnostic test, particularly to run a lateral flow test.

4. Sex toy (1) according to one of the preceding claims, **characterized in that** the test module (8) is fitted modular and/or replaceable inside the housing (2).

5. Sex toy (1) according to one of the preceding claims, **characterized in that** the test module (8) is equipped with at least one reservoir (10) for liquids and/or reagents, particularly diluents, extraction reagents and/or chemical or biological agents for analysing said sample.

6. Sex toy (1) according to one of the preceding claims, **characterized in that** the test module (8) comprises at least one reaction chamber (11) adapted to run at least one process and/or reaction and/or separation with the liquids and/or reagents in contact with said sample, preferably one or more incubation steps and/or binding reactions and/or chromatography.

7. Sex toy (1) according to one of the preceding claims, **characterized in that** the sex toy (1) comprises at least one pump (13) for aspirating a sample through the sampling port (3) and the sampling channel (4), preferably to said reaction chamber.

8. Sex toy (1) according to one of the preceding claims, **characterized in that** the sex toy (1) comprises a microfluidic device (15) to exploit the physical and/or chemical properties of body fluid and/or cells.

9. Sex toy (1) according to one of the preceding claims, **characterized in that** the sampling channel (4) is either moulded integrally to the sampling port (3) of the housing (2) or it is part of the test module (8).

10. Sex toy (1) according to one of the preceding claims, **characterized in that** the housing (2) comprises an embedded window (25) to provide a sight on a diagnostic test performed by the test module (8).

11. Assembly comprising the sex toy of claim 1 and a station (26), preferably a docking station, for analysing diagnostic tests of body fluid and/or cells using the sex toy (1) of claim 1, wherein the station (26) comprises a port (28) for the insertion of the sex toy (1) or at least a test module (8) of said sex toy (1) wherein said station (26) comprises an evaluation unit (33) for the evaluation of diagnostic measurement results either performed by the sex toy (1) or by the docking station (26)

12. Assembly according to claim 11, **characterized in that** the docking station (26) further comprises an analysing unit (32) adapted to operate and/or carry out a diagnostic test, especially analysing and/or screening and/or monitoring one or more diagnostic parameters of the sample body fluid and/or cells collected by said sex toy (1).

13. Assembly according to claim 11 or 12, **characterized in that** the docking station (26) comprises a modular test module (27), preferably inserted into the port for insertion (28) of the docking station of the assembly.

14. Assembly according to one of the preceding claims, **characterized in that** the analysing unit (26) of the docking station comprises a cleaning unit (29) for the cleaning of the sex toy (1), especially the sampling port (3) and/or the sampling channel (4).

15. A method for analysing diagnostic tests of body fluid and/or cells using the sex toy (1) and/or an assembly according to one of the preceding claims, the method comprising
i. Providing the sex toy (1);
ii. Collection of a sample of body fluid and/or cells through the sampling port (3) into the sampling channel (4)
**characterized in that** a diagnostic test is performed by the sex toy and/or by the docking station with said collected sample.
